# EUROPEAN PATENT APPLICATION

(11) **EP 2 468 397 A1**
(43) Date of publication of application: **27.06.2012**
(21) Application number: 10810037.1
(22) Date of filing: 20.08.2010
(51) Int. Cl.: B01J 20/22, B01J 20/34, B01J 23/96, B01J 38/00, C07D 285/00, C22B 3/24, C22B 11/00, C22B 7/00

(54) **PALLADIUM ION ADSORBENT AND METHOD FOR SEPARATING AND RECOVERING PALLADIUM USING SAME**

(30) Priority: 21.08.2009 JP 2009192466; 21.08.2009 JP 2009192467
(71) Applicant: Tosoh Corporation, Yamaguchi 746-8501 (JP)
(72) Inventor: SUDO, Yukinori, Shunan-shi Yamaguchi 746-8501 (JP)
(74) Representative: Kügele, Bernhard
(86) International application number: PCT/JP2010/064104
(87) International publication number: WO 2011/021696

(57) **Abstract**

To provide a solid-liquid palladium ion adsorbent which has both extraction performance and selectivity of a liquid-liquid palladium extracting agent in a conventional solvent extraction method and which does not require use of an organic solvent, and methods for selectively separating and recovering palladium, using it.

Palladium is selectively separated and recovered by using a palladium ion adsorbent comprising an amide-containing cyclic sulfide compound represented by the following formula (1) or (2), or a palladium ion adsorbent having the amide-containing cyclic sulfide compound represented by the following formula (1) or (2) fixed on a carrier: 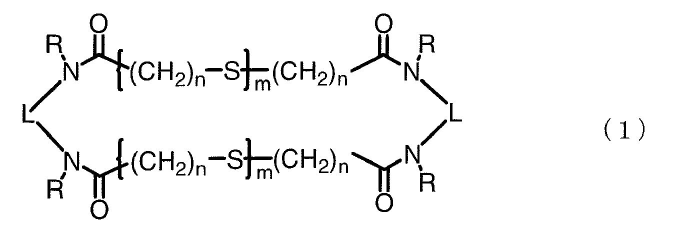 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, m is an integer of 1 or 2, n is each independently an integer of from 1 to 12, and L is each independently a methylene group, an ethylene group, a C₃₋₈ alkylene group or a C₆₋₁₄ arylene group; 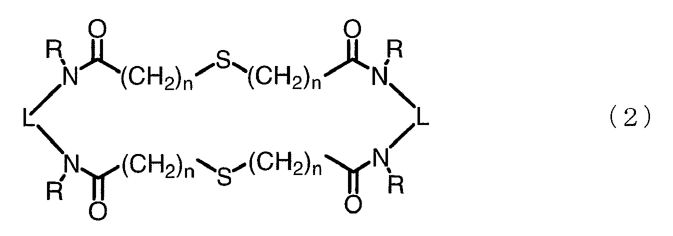 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, n is each independently an integer of from 1 to 4, and L is each independently a methylene group, an ethylene group, a C₃₋₈ alkylene group or a C₆₋₁₄ arylene group.

## Description

### TECHNICAL FIELD

The present invention relates to a palladium ion adsorbent comprising a specific amide-containing cyclic sulfide compound, a palladium ion adsorbent having the amide-containing cyclic sulfide compound fixed on a carrier, and methods for separating and recovering palladium using it.

### BACKGROUND ART

Palladium has been used for industrial catalysts, automobile exhaust gas purifying catalysts and many electric appliances. Since palladium is expensive and is useful as resources, it has been recovered after use recycled. In recent years, in view of the resources conservation, importance of recovery and recycle is more increasing.

To recover palladium, many methods such as a sedimentation separation method, an ion exchange method, an electrodeposition method and a solvent extraction method have been developed, and among them, a solvent extraction method has been widely employed in view of the economical efficiency and operation properties. For example, a method has been known wherein by liquid-liquid contact of palladium ions in an aqueous solution with an organic solvent having an oil-soluble extracting agent dissolved, the palladium ions are extracted into the organic phase side. As the extracting agent, a sulfur-containing organic compound such as a dialkyl sulfide has been used (for example, Patent Document 1). Further, in order to improve the extraction rate, a method of introducing an amide group to the vicinity of sulfur in the dialkyl sulfide has been proposed (for example, Patent Document 2).

However, the above-described solvent extraction method has problems in view of the safety and the environmental burden, since a large quantity of an organic solvent is used.

Accordingly, a method (adsorption method) using no organic solvent, wherein a specific thioether is used as a ligand and fixed on a polymer (for example, polymethylstyrene) of a styrene derivative to obtain a water-insoluble solid polymer sulfide compound, and this compound is directly added to an aqueous solution containing palladium ions to carry out adsorption of the palladium ions (for example, Patent Document 3).

However, the adsorption method disclosed in Patent Document 3 had a problem such that the palladium adsorption amount of the adsorbent was so low as 4 mg (0.04 mmol) per 1 g of the adsorbent.

As described above, to recover palladium, the solvent extraction method has been widely employed in view of economical efficiency and the operation properties, but it has a problem such that use of an organic solvent is essential.

On the other hand, the adsorption method employing a water-insoluble polymer chelating agent or an ion exchange resin has advantages such that metal separation can be carried out without using an organic solvent, but provides a low selectivity of a specific metal in many cases. For example, a finding that palladium is selectively separated from a solution in which a plurality of metals are present, by the polymer sulfide compound disclosed in Patent Document 3 is not disclosed in Patent Document 3.

### PRIOR ART DOCUMENTS

### PATENT DOCUMENTS

Patent Document 1: JP-A-9-279264
Patent Document 2: W02005/083131
Patent Document 3: JP-A-5-105973

### DISCLOSURE OF INVENTION

### TECHNICAL PROBLEM

Under these circumstances, it is an object of the present invention to provide a solid-liquid palladium adsorbent which has both extraction performance and selectivity of a liquid-liquid palladium extracting agent in a conventional solvent extraction method and which does not require use of an organic solvent, and methods for selectively separating and recovering palladium using it.

### SOLUTION TO PROBLEM

The present inventor has been conducted extensive studies to achieve the above object and as a result, found an adsorbent comprising a specific amide-containing cyclic sulfide compound and an adsorbent having the amide-containing cyclic sulfide compound supported on a porous carrier. He has further found that the above object can be achieved by carrying out separation and recovery of palladium by using such an adsorbent. The present invention has been accomplished on the basis of these discoveries.

That is, the present invention provides the following palladium ion adsorbent and methods for separating and recovering palladium using it.
[1] A palladium ion adsorbent, comprising an amide-containing cyclic sulfide compound represented by the following formula (1): 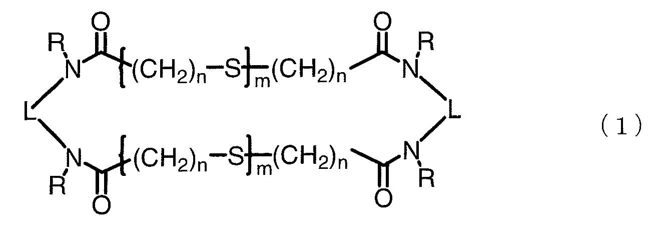 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, m is an integer of 1 or 2, n is each independently an integer of from 1 to 12, and L is each independently a methylene group, an ethylene group, a C₃₋₈ alkylene group or a C₆₋₁₄ arylene group.
[2] The palladium ion adsorbent according to the above [1], wherein in the formula (1), R is each independently a hydrogen atom, a methyl group, an ethyl group or a C₃₋₁₈ linear or branched chain hydrocarbon group, and n between the carbonyl group and the sulfur atom is each independently an integer of from 1 to 4.
[3] A palladium ion adsorbent, comprising an amide-containing cyclic sulfide compound represented by the following formula (2): 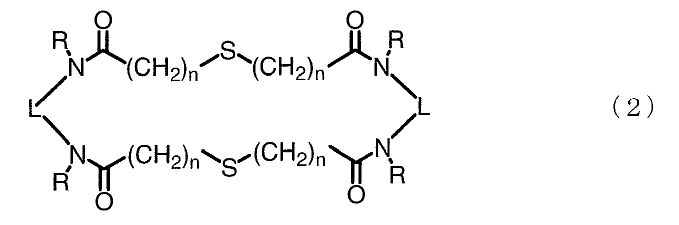 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, n is each independently an integer of from 1 to 4, and L is each independently a methylene group, an ethylene group, a C₃₋₈ alkylene group or a C₆₋₁₄ arylene group.
[4] The palladium ion adsorbent according to the above [3], wherein in the formula (2), R is each independently a hydrogen atom, a methyl group, an ethyl group or a C₃₋₁₈ linear or branched chain hydrocarbon group.
[5] A palladium ion adsorbent, having an amide-containing cyclic sulfide compound represented by the following formula (1) or an amide-containing cyclic sulfide compound represented by the following formula (2) fixed on a carrier: 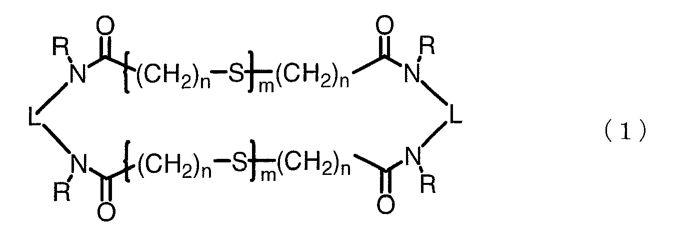 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, m is an integer of 1 or 2, n is each independently an integer of from 1 to 12, and L is each independently a methylene group, an ethylene group, a C₃₋₈ alkylene group or a C₆₋₁₄ arylene group; 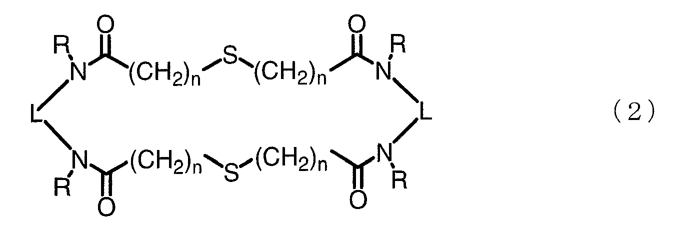 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, n is each independently an integer of from 1 to 4, and L is each independently a methylene group, an ethylene group, a C₃-₈ alkylene group or a C₆₋₁₄ arylene group.
[6] The palladium ion adsorbent according to the above [5], wherein in the formula (1), R is each independently a hydrogen atom, a methyl group, an ethyl group or a C₃₋₁₈ linear or branched chain hydrocarbon group, and n between the carbonyl group and the sulfur atom is each independently an integer of from 1 to 4.
[7] The palladium ion adsorbent according to the above [5], wherein in the formula (2), R is each independently a hydrogen atom, a methyl group, an ethyl group or a C₃₋₁₈ linear or branched chain hydrocarbon group.
[8] The palladium ion adsorbent according to any one of the above [5] to [7], wherein the carrier is silica gel.
[9] A method for separating palladium, which comprises bringing the palladium ion adsorbent as defined in any one of the above [1] to [8] into contact with an aqueous solution containing palladium to make palladium be adsorbed in the palladium ion adsorbent.
[10] A method for recovering palladium, which comprises bringing the palladium ion adsorbent as defined in any one of the above [1] to [8] into contact with an aqueous solution containing palladium to make palladium be adsorbed in the palladium ion adsorbent, and then eluting palladium adsorbed in the palladium ion adsorbent by an eluent to obtain an aqueous solution containing palladium.

### ADVANTAGEOUS EFFECTS OF INVENTION

The palladium ion adsorbent of the present invention (hereinafter sometimes referred to as "the adsorbent of the present invention") is an amide-containing cyclic sulfide compound itself represented by the above formula (1); an amide-containing cyclic sulfide compound itself represented by the above formula (2); one having an amide-containing cyclic sulfide compound represented by the above formula (1) fixed on a carrier; or one having an amide-containing cyclic sulfide compound represented by the above formula (2) fixed on a carrier.

The amide-containing cyclic sulfide compound represented by the above formula (1) or (2) is solid, differently from a conventional oily sulfide type palladium extracting agent such as a dialkyl sulfide, and has extremely low water solubility and accordingly, it can be directly added to an aqueous solution containing palladium ions without being dissolved in an organic solvent to carry out adsorption of palladium ions.

Further, the palladium ion adsorbent of the present invention has a high affinity with palladium, whereby in a case where a plurality of ions of platinum group metals such as platinum and rhodium are present in addition to palladium, particularly the palladium ions can be highly selectively adsorbed.

Further, according to the method for separating or recovering palladium of the present invention, palladium in an aqueous solution containing palladium ions can efficiently and selectively be adsorbed in the palladium ion adsorbent of the present invention, and further by using an eluent, palladium adsorbed in the adsorbent can efficiently be recovered.

Further, according to the method for separating or recovering palladium of the present invention, palladium in industrial catalysts or automobile exhaust gas purifying catalysts can efficiently and selectively be adsorbed and recovered without using an organic solvent.

### BRIEF DESCRIPTION OF DRAWINGS

Fig. 1 is a graph illustrating the relation between the amide-containing sulfide amount in the palladium ion adsorbent and the metal adsorption ratio in Example 1.
Fig. 2 is a graph illustrating the relation between the amide-containing sulfide amount in the palladium ion adsorbent and the metal adsorption ratio in Example 7.

### DESCRIPTION OF EMBODIMENTS

In the present invention, palladium to be adsorbed, separated, recovered, extracted or the like, is in the form of ions in a solution to be treated. Accordingly, in this specification, "palladium" sometimes means "palladium ions".

First, the amide-containing cyclic sulfide compound represented by the above formula (1) will be described.

In the above formula (1), each of substituents represented by R which are independent of each other, is a hydrogen atom, a methyl group, an ethyl group, a C₃₋₃₀ linear or branched chain hydrocarbon group (such a group may be branched), a C₃₋₁₀ alicyclic hydrocarbon group or a C₆₋₁₄ aromatic hydrocarbon group. Among them, preferred is a hydrogen atom, a methyl group, an ethyl group, a C₃₋₁₈ linear or branched chain hydrocarbon group, a C₅₋₈ alicyclic hydrocarbon group or a C₆₋₈ aromatic hydrocarbon group. In view of the palladium adsorption performance, more preferred is a compound wherein all the substituents represented by R are hydrogen atoms. Further, when m=1, in view of the durability as the palladium ion adsorbent, more preferred is a compound wherein at least one substituent represented by R is the above-described methyl group, ethyl group, C₃₋₁₈ linear or branched chain hydrocarbon group, C₅₋₈ alicyclic hydrocarbon group or C₆₋₈ aromatic hydrocarbon group.

The C₃₋₃₀ linear or branched chain hydrocarbon group may, for example, be a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group (cetyl group), a heptadecyl group (stearyl group), an octadecyl group, an oleyl group, a nonadecyl group, an eicosyl group, an isopropyl group, an isobutyl group, a s-butyl group, a t-butyl group, an isopentyl group, a neopentyl group, a t-pentyl group, a 2-ethylhexyl group, a vinyl group, an allyl group, a 1-propenyl group, an isopropenyl group, a 1-butenyl group, a 2-butenyl group, a 2-methylallyl group, a 1-heptynyl group, a 1-hexenyl group, a 1-heptenyl group, a 1-octenyl group or a 2-methyl-1-propenyl group.

The C₃₋₁₀ alicyclic hydrocarbon group may, for example, be a cyclopropyl group, a cyclobutyl group, a cyclopentyl group, a cyclohexyl group, a cycloheptyl group, a cyclooctyl group, a cyclononyl group, a cyclodecyl group, a cyclohexenyl group, a cyclohexadienyl group, a cyclohexatrienyl group, a cyclooctenyl group or a cyclooctadienyl group.

The C₆₋₁₄ aromatic hydrocarbon group may, for example, be a phenyl group, a naphthyl group, an anthryl group, a tolyl group, a xylyl group, a cumenyl group, a benzyl group, a phenethyl group, a styryl group, a cinnamyl group, a biphenylyl group or a phenanthryl group.

In the above formula (1), m is an integer of 1 or 2.

In the above formula (1), n is each independently an integer of from 1 to 12. In the above formula (1), n representing the number of methylene between the carbonyl group and the sulfur atom is preferably an integer of from 1 to 4, particularly preferably 1 or 2.

In the above formula (1), L is each independently a methylene group, an ethylene group, a C₃₋₈ alkylene group or a C₆₋₁₄ arylene group.

The C₃₋₈ alkylene group may, for example, be a propylene group, a butylene group, a pentylene group, a hexylene group, a heptylene group or an octylene group, and it may be linear, branched or cyclic.

The cyclic alkylene group may, for example, be a cyclopropylene group, a cyclobutylene group, a cyclopentylene group, a cyclohexylene group, a cycloheptylene group, a cyclooctylene group, a cyclohexenylene group, a cyclohexadienylene group, a cyclohexatrienylene group, a cyclooctenylene group or a cyclooctadienylene group.

Further, the C₆₋₁₄ arylene group may, for example, be a phenylene group, a naphthylene group, an anthrylene group, a tolylene group, a xylylene group, a cumenylene group, a benzylene group, a phenethylene group, a styrylene group, a cinnamylene group, a biphenylylene group or a phenanthrylene group.

Among them, L is preferably a 1,2-ethylene group, a 1,3-propylene group, a 1,4-butylene group, a 1,2-cyclohexylene group or a 1,2-phenylene group, and the highest palladium adsorption performance can be obtained particularly when it is a 1,3-propylene group.

A process for producing the amide-containing cyclic sulfide compound represented by the above formula (1) is not particularly limited, and a compound wherein m=1 may be produced, for example, as follows.

That is, a compound represented by the following formula (3): 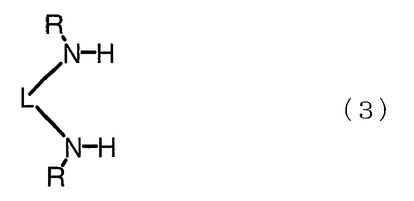 wherein R and L are as defined above, is reacted with a compound represented by the following formula (4): 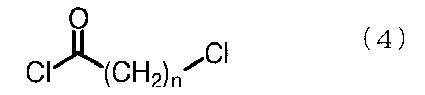 wherein n is as defined above, under basic conditions to obtain a compound represented by the following formula (5): 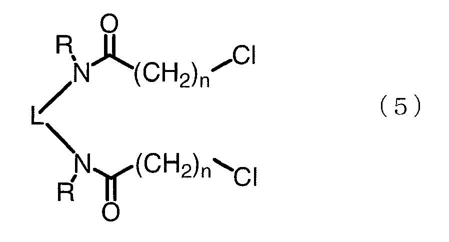 wherein R, L and n are as defined above, the compound represented by the above formula (5) is reacted with thiobenzoic acid under basic conditions to obtain a compound represented by the following formula (6): 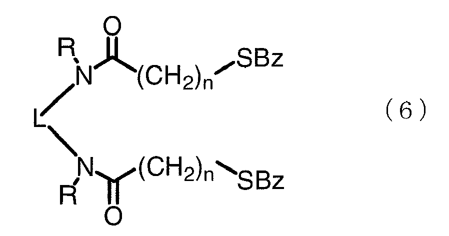 wherein Bz is a benzoyl group, and R, L and n are as defined above, and then the compound represented by the above formula (5) is reacted with the compound represented by the above formula (6) under basic conditions to obtain the amide-containing cyclic sulfide compound (m=1) represented by the above formula (1).

Further, among the amide-containing cyclic sulfide compounds represented by the above formula (1), a compound wherein m=1 may be produced, for example, as follows. That is, the compound represented by the above formula (6) prepared as mentioned above is reacted with a compound represented by the following formula (7): 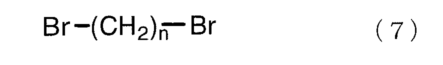 wherein n is as defined above, under basic conditions to obtain the amide-containing cyclic sulfide compound (m=2) represented by the above formula (1).

The amide-containing cyclic sulfide compound represented by the above formula (1) is solid and has extremely low water solubility, and thus it can be added as it is to an aqueous solution containing palladium ions to carry out adsorption. In a case where ions of other platinum group metals such as platinum ions and rhodium ions are present in the aqueous solution, the palladium ions are adsorbed with high selectivity.

Now, the amide-containing cyclic sulfide compound represented by the above formula (2) will be described below.

In the above formula (2), each of substituents represented by R which are independent of each other, is a hydrogen atom, a methyl group, an ethyl group, a C₃₋₃₀ linear or branched chain hydrocarbon group (such a group may be branched), a C₃₋₁₀ alicyclic hydrocarbon group or a C₆₋₁₄ aromatic hydrocarbon group. Among them, preferred is a hydrogen atom, a methyl group, an ethyl group, a C₃₋₁₈ linear or branched chain hydrocarbon group, a C₅₋₈ alicyclic hydrocarbon group or a C₆₋₈ aromatic hydrocarbon group. In view of the palladium adsorption performance, more preferred is a compound wherein all the substituents represented by R are hydrogen atoms. Further, in view of the durability as the palladium ion adsorbent, more preferred is a compound wherein at least one substituent represented by R is the above-described methyl group, ethyl group, C₃₋₁₈ linear or branched chain hydrocarbon group, C₅₋₈ alicyclic hydrocarbon group or C₆₋₈ aromatic hydrocarbon group.

The C₃₋₃₀ linear or branched chain hydrocarbon group may, for example, be a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group (cetyl group), a heptadecyl group (stearyl group), an octadecyl group, an oleyl group, a nonadecyl group, an eicosyl group, an isopropyl group, an isobutyl group, a s-butyl group, a t-butyl group, an isopentyl group, a neopentyl group, a t-pentyl group, a 2-ethylhexyl group, a vinyl group, an allyl group, a 1-propenyl group, an isopropenyl group, a 1-butenyl group, a 2-butenyl group, a 2-methylallyl group, a 1-heptynyl group, a 1-hexenyl group, a 1-heptenyl group, a 1-octenyl group or a 2-methyl-1-propenyl group.

The C₃₋₁₀ alicyclic hydrocarbon group may, for example, be a cyclopropyl group, a cyclobutyl group, a cyclopentyl group, a cyclohexyl group, a cycloheptyl group, a cyclooctyl group, a cyclononyl group, a cyclodecyl group, a cyclohexenyl group, a cyclohexadienyl group, a cyclohexatrienyl group, a cyclooctenyl group or a cyclooctadienyl group.

The C₆₋₁₄ aromatic hydrocarbon group may, for example, be a phenyl group, a naphthyl group, an anthryl group, a tolyl group, a xylyl group, a cumenyl group, a benzyl group, a phenethyl group, a styryl group, a cinnamyl group, a biphenylyl group or a phenanthryl group.

In the above formula (2), the number of methylene between the carbonyl group and the sulfur atom represented by n is preferably an integer of from 1 to 4, particularly preferably 1 or 2.

In the above formula (2), L is a C₁₋₈ alkylene group or a C₆₋₁₄ arylene group.

The C₁₋₈ alkylene group may, for example, be a methylene group, an ethylene group, a propylene group, a butylene group, a pentylene group, a hexylene group, a heptylene group or an octylene group, and it may be linear, branched or cyclic.

The cyclic alkylene group may, for example, be a cyclopropylene group, a cyclobutylene group, a cyclopentylene group, a cyclohexylene group, a cycloheptylene group, a cyclooctylene group, a cyclohexenylene group, a cyclohexadienylene group, a cyclohexatrienylene group, a cyclooctenylene group or a cyclooctadienylene group.

Further, the C₆₋₁₄ arylene group may, for example, be a phenylene group, a naphthylene group, an anthrylene group, a tolylene group, a xylylene group, a cumenylene group, a benzylene group, a phenethylene group, a styrylene group, a cinnamylene group, a biphenylylene group or a phenanthrylene group. Among them, L is preferably a 1,2-ethylene group, a 1,3-propylene group, a 1,4-butylene group, a 1,2-cyclohexylene group or a 1,2-phenylene group, and the highest palladium adsorption performance can be obtained particularly when it is a 1,3-propylene group.

A process for producing the amide-containing cyclic sulfide compound represented by the above formula (2) is not particularly limited, and for example, it can be produced in the same manner as for the above-described amide-containing cyclic sulfide compound (m=1) represented by the above formula (1).

The amide-containing cyclic sulfide compound represented by the above formula (2) is solid and has extremely low water solubility, and thus it can be added as it is to an aqueous solution containing palladium ions to carry out adsorption. In a case where ions of other platinum group metals such as platinum ions and rhodium ions are present in the aqueous solution, the palladium ions are adsorbed with high selectivity.

Now, the palladium ion adsorbent having the amide-containing cyclic sulfide compound represented by the above formula (1) or the amide-containing cyclic sulfide compound represented by the above formula (2) fixed on a carrier will be described.

In the present invention, the amide-containing cyclic sulfide compound represented by the above formula (1) and the amide-containing cyclic sulfide compound represented by the above formula (2) can be used as they are as a palladium ion adsorbent, or they may be fixed on an optional carrier depending on purposes such as improvement in the operation properties or use as a filler of column chromatography.

In the present invention, the carrier is not particularly limited so long as it is insoluble in water. It may, for example, be a polymer carrier such as a styrene polymer such as polystyrene or crosslinked polystyrene; a polyolefin such as polyethylene or polypropylene; a poly(halogenated olefin) such as polyvinyl chloride or polytetrafluoroethylene; a nitrile polymer such as polyacrylonitrile; or a (meth)acrylic acid polymer such as polymethyl methacrylate or polyethyl acrylate, or an inorganic carrier such as activated carbon, silica gel, diatomaceous earth, hydroxyapatite, alumina, titanium oxide, magnesia or a polysiloxane.

The crosslinked polystyrene is one comprising as the main component a crosslinked copolymer of a monovinyl aromatic compound such as styrene, vinyltoluene, vinylxylene or vinylnaphthalene with a polyvinyl aromatic compound such as divinylbenzene, divinyltoluene, divinylxylene, divinylnaphthalene, trivinylbenzene, bisvinyldiphenyl or bisvinylphenylethane, and with such a copolymer, a methacrylate ester such as glycerol methacrylate or ethylene glycol dimethacrylate may be copolymerized.

In the present invention, among such carriers, silica gel is particularly preferred.

As the shape of the carrier to be used in the present invention, shapes commonly employed as a separation substrate such as spheres (for example, spherical particles), particles, fibers, granules, monolithic columns, hollow fibers and a film (for example, a flat membrane) may be used, and the shape is not particularly limited. Among them, spheres, a film, particles or fibers are preferred. Spherical particles are particularly preferably used, since the volume of their use can be flexibly set, when used in a column method or a batch method.

In a case where spherical particles are used as the carrier, the average particle size is usually within a range of from 1 µm to 10 mm, preferably within a range of from 2 µm to 1 mm, and the average pore size is usually within a range of from 1 nm to 1 µm, preferably within a range of from 1 nm to 300 nm.

In such a case, a method of fixing the amide-containing cyclic sulfide compound represented by the above formula (1) or the amide-containing cyclic sulfide compound represented by the above formula (2) on the carrier is not particularly limited. It may, for example, be a method of physically making the amide-containing cyclic sulfide compound represented by the above formula (1) or the amide-containing cyclic sulfide compound represented by the above formula (2) be adsorbed in and supported on the carrier, or a method of chemically binding the amide-containing cyclic sulfide compound represented by the above formula (1) or the amide-containing cyclic sulfide compound represented by the above formula (2) with the carrier to make the compound be fixed.

The adsorbent of the present invention can be produced by dissolving the amide-containing cyclic sulfide compound represented by the above formula (1) or the amide-containing cyclic sulfide compound represented by the above formula (2) in a solvent such as dimethyl sulfoxide, then adding the above carrier to impregnate the carrier with the amide-containing cyclic sulfide compound, and further distilling the solvent off.

As a solvent used, in addition to dimethyl sulfoxide, methanol, ethyl acetate, tetrahydrofuran or the like may be mentioned as a preferred example.

Further, for example, in a case where the carrier is a crosslinked polystyrene, polychloromethylstyrene (PCMS) which is a crosslinked polystyrene of chloromethylstyrene with divinylbenzene, and the amide-containing cyclic sulfide compound represented by the above formula (1) or the amide-containing cyclic sulfide compound represented by the above formula (2) are chemically bonded by a reaction under basic conditions to produce the adsorbent of the present invention.

As a base to carry out the reaction under basic conditions, sodium hydroxide, potassium hydroxide, sodium hydride or the like is preferably used.

In the adsorbent of the present invention, the fixation ratio (support ratio) of the amide-containing cyclic sulfide compound on the carrier may optionally be adjusted depending upon the purpose of use and is not particularly limited. However, it is preferred that the amide-containing cyclic sulfide compound represented by the above formula (1) or the amide-containing cyclic sulfide compound represented by the above formula (2) is fixed (supported) within a range of from 1 to 50 wt%, more preferably within a range of from 5 to 30 wt%, based on the adsorbent of the present invention.

Now, the method for separating palladium of the present invention will be described.

The method for separating palladium of the present invention is characterized by bringing the palladium ion adsorbent of the present invention into contact with an aqueous solution containing palladium to make palladium be adsorbed in the palladium ion adsorbent.

In the method for separating palladium of the present invention, adsorption of palladium with good efficiency is carried out at a concentration of platinum group metals (such as palladium, platinum and rhodium) including palladium in the aqueous solution of from 1 to 10,000 ppm, more preferably from 10 to 1,000 ppm.

Further, the method for recovering palladium of the present invention is characterized by bringing the palladium ion adsorbent of the present invention into contact with an aqueous solution containing palladium to make palladium be adsorbed in the adsorbent, and then eluting palladium adsorbed in the adsorbent by an eluent to obtain an aqueous solution containing palladium.

In the methods for separating and recovering palladium, the solution to be treated may, for example, be an aqueous solution having an automobile exhaust gas treating catalyst dissolved or a solution after acid leaching in a step of wet refining of platinum group metals. Such a solution to be treated contains platinum group metals such as palladium, platinum and rhodium, however, the components other than palladium are not essential.

As the form of the platinum group metals contained in the solution to be treated, they are contained in the solution in a state of e.g. bivalent metal ions.

To adsorb palladium in the adsorbent of the present invention, first, the adsorbent of the present invention is added to the above solution to be treated. At this time, the solution is preferably stirred. Further, the solution to be treated is preferably acidic, more preferably acidic by hydrochloric acid. The hydrochloric acid concentration in the solution to be treated is not particularly limited as the adsorbent of the present invention can be used within a wide hydrochloric acid concentration range, but is preferably within a range of from 0.1 to 5 mol/L, more preferably within a range of from 0.5 to 3 mol/L. Within such a hydrochloric acid concentration range, adsorption can be carried out without impairing the efficiency of adsorption of palladium.

Further, in the above-described methods for separating and recovering palladium, it is preferred to use the adsorbent of the present invention in an equimolar amount or more, as calculated as the amide-containing cyclic sulfide compound represented by the above formula (1), relative to palladium in the solution to be treated.

Palladium adsorbed in the adsorbent of the present invention by the above-described operation is eluted by means of an eluent to obtain an aqueous solution containing palladium, thereby to recover palladium. The eluent for palladium is not particularly limited, and for example, ammonia water, a thiourea aqueous solution or a mixed aqueous solution of a thiourea aqueous solution with hydrochloric acid may be preferably used. Further, an ethylenediamine aqueous solution can also be preferably used as an eluent for palladium.

With respect to the amounts of the palladium ion adsorbent in which palladium is adsorbed and the eluent, the amount of the eluent is preferably from 50 to 1,000 molar times, more preferably from 100 to 500 molar times, the amount of the adsorbent.

When palladium is adsorbed by using the adsorbent of the present invention, palladium can be recovered as an aqueous solution by using the above eluent.

### EXAMPLES

Now, the present invention will be described in detail with reference to Examples. However, it should be understood that the present invention is by no means restricted to such specific Examples.

In the following Examples, ¹H-NMR (nuclear magnetic resonance) was measured by Gemini-200 manufactured by Varian.

Further, in Examples, the unit "mmol/g-Ligand" for the palladium adsorption amount means the number of mols of palladium adsorbed per 1 g of Ligand, and "Ligand" means the amide-containing cyclic sulfide compound of this invention.

### PREPARATION EXAMPLE 1

As an example for preparation of the amide-containing cyclic sulfide compound, an example for preparation of 1,5,11,15-tetraaza-6,10,16,20-tetraoxo-8,18-dithiacycloeicosane (hereinafter referred to as compound (8)) will be described below.

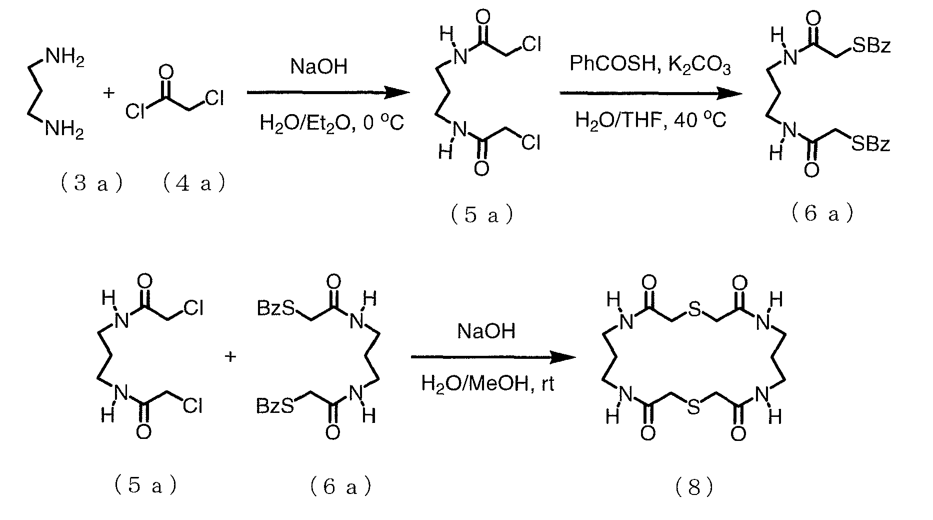

### FIRST STEP (preparation of diacylated form (5a))

In a 200 mL eggplant flask, 3.71 g (50 mmol) of 1,3-propanediamine (3a), 50 g of water and 20 g of diethyl ether were weighed and 24.00 g (120 mmol) of a 20 wt% sodium hydroxide aqueous solution was added. To the mixture, 13.55 g (120 mmol) of chloroacetyl chloride (4a) was dropwise added at 0°C over a period of one hour, followed by stirring at 0°C for one hour. The formed white solid was collected by filtration and sequentially washed with water and diethyl ether to obtain a diacylated form represented by the above formula (5a) (hereinafter referred to as diacylated form (5a)) in an amount of 10.41 g with a yield of 91.7%.

¹H-NMR (DMSO-d₆) δ: 1.55 (2H, quintet, J=7.0 Hz), 3.03 to 3.10 (4H, m), 4.03 (4H, s), 8.22 (2H, brs)

### SECOND STEP (preparation of dithioesterified form (6a))

In a 100 mL eggplant flask, 2.65 g (19.2 mmol) of potassium carbonate and 40 g of water were weighed, and 2.65 g (19.2 mmol) of thiobenzoic acid was added, followed by stirring at 40°C for 30 minutes. To the mixture, 1.82 g (8 mmol) of the above diacylated form (5a) and 10 g of tetrahydrofuran (THF) were added, followed by stirring at 40°C for 3 hours. Then, stirring was carried out at 0°C further for one hour, and the formed white solid was collected by filtration and washed with water to obtain a dithioesterified form represented by the above formula (6a) (hereinafter referred to as a dithioesterified form (6a)) in an amount of 3.51 g with a yield of 95.6%.

¹H-NMR (CDCl₃) δ: 1.65 (2H, quintet, J=6.2 Hz), 3.23 to 3.32 (4H, m), 3.74 (4H, s), 6.88 (2H, brs), 7.43 to 7.52 (4H, m), 7.57 to 7.66 (2H, m), 7.95 to 8.00 (4H, m) THIRD STEP (preparation of compound (8))

In a 50 mL eggplant flask, 2.15 g (5 mmol) of the dithioesterified form (6a) and 20 g of methanol were weighed, and 2.00 g (10 mmol) of a 20 wt% sodium hydroxide aqueous solution was added, followed by stirring in a stream of nitrogen at room temperature for 2 hours. To the mixture, 1.14 g (5 mmol) of the above diacylated form (5a) was added, followed by stirring at room temperature for 3 hours. The formed white solid was collected by filtration and sequentially washed with water and methanol to obtain an amide-containing cyclic sulfide compound represented by the above formula (8) (hereinafter referred to as compound (8)) in an amount of 1.43 g with a yield of 76.1%.

¹H-NMR (DMSO-d₆) δ: 1.50 to 1.57 (4H, m), 3.01 to 3.10 (8H, m), 3.19 (8H, s), 8.05 (4H, brs)

### PREPARATION EXAMPLE 2

In the same manner as in Preparation Example 1 except that in the First step, 3.01 g of 1,2-ethylenediamine was used instead of 3.71 g of 1,3-propanediamine, an amide-containing cyclic sulfide compound represented by the following formula (9): 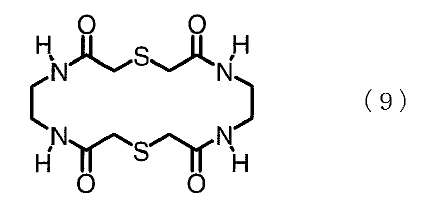 was prepared (amount: 1.74 g, yield from diamine: 71.1 %).

¹H-NMR (DMSO-d₆) δ: 1.34 to 1.42 (8H, m), 3.01 to 3.10 (8H, m), 3.18 (8H, s), 8.03 (4H, brs)

### PREPARATION EXAMPLE 3

In the same manner as in Preparation Example 1 except that in the First step, 4.41 g of 1,4-butanediamine was used instead of 3.71 g of 1,3-propanediamine, an amide-containing cyclic sulfide compound represented by the following formula (10): 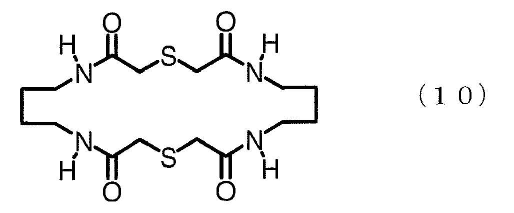 was prepared (amount: 1.96 g, yield from diamine: 78.5%).

¹H-NMR (DMSO-d₆) δ: 1.50to 1.57 (4H, m), 3.01 to 3.10 (8H, m), 3.19 (8H, s), 8.05 (4H, brs)

### PREPARATION EXAMPLE 4

In the same manner as in Preparation Example 1 except that in the First step, 5.71 g of 1,2-cyclohexanediamine was used instead of 3.71 g of 1,3-propanediamine, an amide-containing cyclic sulfide compound represented by the following formula (11): 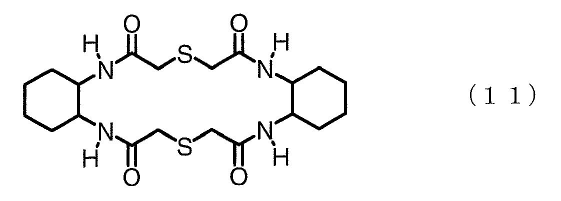 was prepared (amount: 2.26 g, yield from diamine: 89%).

¹H-NMR (DMSO-d₆) δ: 1.17 to 1.28 (8H, m), 1.61 to 1.87 (8H, m), 3.16 (8H, s), 7.86 (4H, brs), undetected 4H (estimated to be overlapping with the peak of water).

### PREPARATION EXAMPLE 5

In the same manner as in Preparation Example 1 except that in the First step, 5.41 g of 1,2-phenylenediamine was used instead of 3.71 g of 1,3-propanediamine, an amide-containing cyclic sulfide compound represented by the following formula (12): 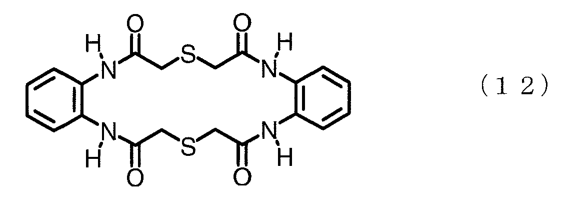 was prepared (amount: 1.88 g, yield from diamine: 75.7%).

¹H-NMR (DMSO-d₆) δ: 7.11 to 7.21 (4H, m), 7.50 to 7.60 (4H, m), 9.59 (4H, brs), undetected 8H (estimated to be overlapping with the peak of water).

### PREPARATION EXAMPLE 6

In the same manner as in Preparation Example 1 except that in the First step, 3.01 g of 1,2-ethylenediamine was used instead of 3.71 g of 1,3-propanediamine and 15.24 g of 3-chloropropionyl chloride was used, an amide-containing cyclic sulfide compound represented by the following formula (13): 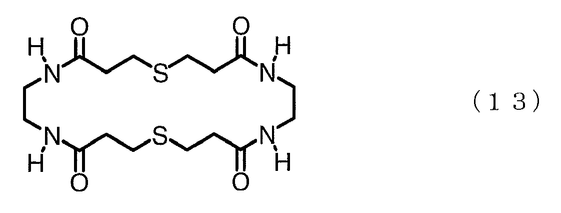 was prepared (amount: 1.95 g, yield from diamine: 55.5%).

¹H-NMR (DMSO-d₆) δ: 2.28 to 2.35 (8H, m), 2.64 to 2.71 (8H, m), 3.08 to 3.15 (8H, m), 7.76 (4H, brs)

### PREPARATION EXAMPLE 7

In the same manner as in Preparation Example 1 except that in the First step, 9.51 g of beef tallow propylenediamine (manufactured by Kao Corporation, trade name: Diamine RRT) and 8.47 g of chloroacetyl chloride were used instead of 3.71 g of 1,3-propanediamine, a diacylated form represented by the following formula (5b): 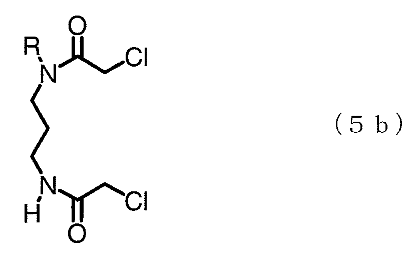 wherein the substituent represented by R is a long chain alkyl group (for example, including a cetyl group, a stearyl group and an oleyl group) derived from the beef tallow, is obtained in an amount of 12.59 g with a yield of 89.3%.

Then, in the Third step, in the same manner as in Preparation Example 1 except that 12.58 g of the above diacylated form (5b) and 11.53 g of the dithioesterified form (6a) were used instead of 1.14 g of the diacylated form (5a), an amide-containing cyclic sulfide compound represented by the following formula (14): 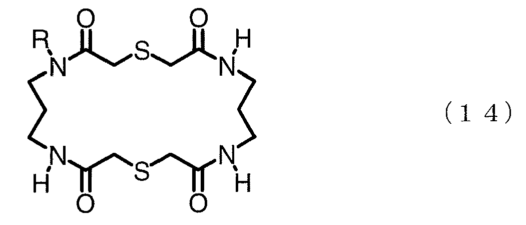 wherein the substituent represented by R is a long chain alkyl group (for example, including a cetyl group, a stearyl group and an oleyl group) derived from the beef tallow, was obtained in an amount of 15.40 g with a yield of 92.9% (in the above formula (14), the substituent represented by R is a long chain alkyl group (such as a cetyl group, a stearyl group or an oleyl group) derived from the beef tallow).

¹H-NMR (CDCl₃) δ: 0.85 to 0.91 (m, peak attributable to the beef tallow alkyl group), 1.25 to 1.31 (m, peak attributable to the beef tallow alkyl group), 1.50 to 2.03 (m, peak attributable to the beef tallow alkyl group), 3.26 to 3.52 (m, 18H), 5.32 to 5.38 (m, peak attributable to the beef tallow alkyl group), 7.36 (1H, brs), 7.58 (1H, brs), 7.70 (1H, brs), undetected 4H (estimated to be overlapping with the peak attributable to the beef tallow alkyl group).

### PREPARATION EXAMPLE 8

In the same manner as in Preparation Example 1 except that in the Third step, 1.43 g of 1,6-dibromohexane was used instead of 1.14 g of the diacylated form (5a), an amide-containing cyclic sulfide compound represented by the following formula (15): 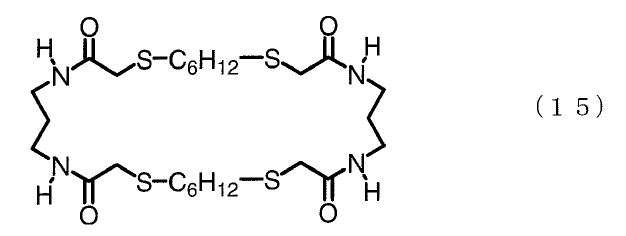 was prepared (amount: 1.16 g, yield from diamine: 70.0%).

¹H-NMR (DMSO-d₆) δ: 1.34 to 1.39 (8H, m), 1.50 to 1.63 (12H, m), 2.55 to 2.61 (8H, m), 3.05 to 3.17 (16H, m), 8.00 to 8.04 (4H, brs)

### PREPARATION EXAMPLE 9

In the same manner as in Preparation Example 1 except that in the Third step, 1.43 g of 1,9-dibromononane was used instead of 1.14 g of the diacylated form (5a), an amide-containing cyclic sulfide compound represented by the following formula (16): 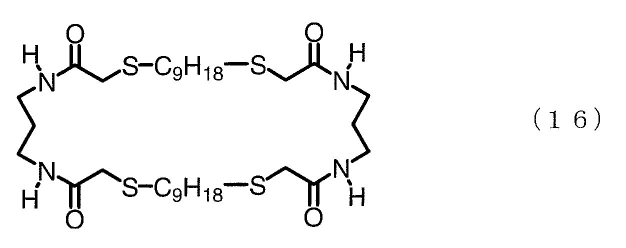 was prepared (amount: 1.34 g, yield from diamine: 71.1 %).

¹H-NMR (CDCl₃) δ: 1.28 to 1.39 (20H, m), 1.53 to 1.80 (12H, m), 2.51 to 2.58 (8H, m), 3.23 (8H, s), 3.29 to 3.41 (8H, m), 7.36 (4H, brs)

### EXAMPLE 1

10 mg of the amide-containing cyclic sulfide compound prepared in Preparation Example 1 was added to 10 mL of a 1 mol/L hydrochloric acid solution containing 50 mg/L each of palladium, platinum and rhodium, followed by stirring at room temperature for one hour. Then, the solution was subjected to filtration through a membrane filter having a pore size of 0.45 µm, and the metal concentration remaining in the filtrate was measured by an ICP emission spectrometer (manufactured by PerkinElmer Co., Ltd., trade name: OPTIMA 3300 DV). The adsorption ratio of each metal was determined from the remaining metal concentration and the initial concentration before stirring and as a result, the palladium adsorption ratio was 96.0%, the platinum adsorption ratio was 7.0% and the rhodium adsorption ratio was 0%, thus indicating highly selective adsorption of palladium.

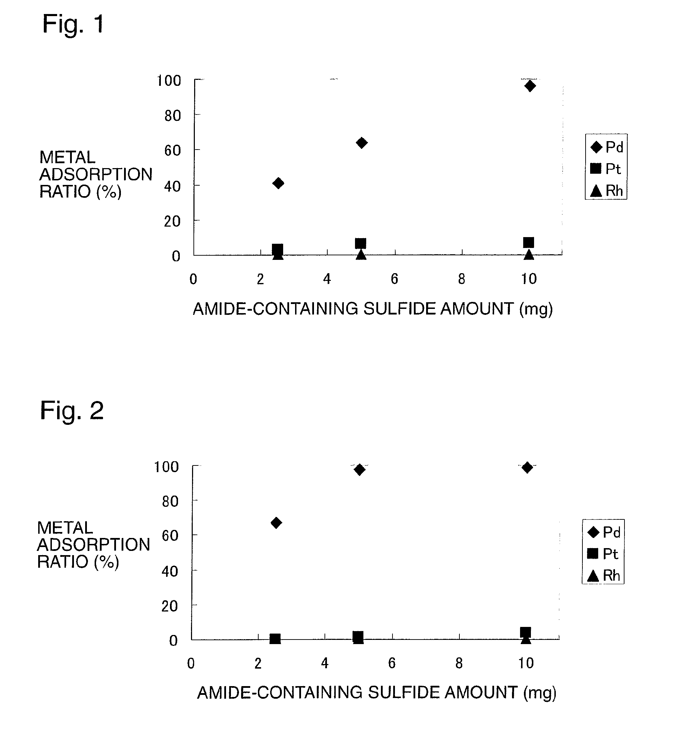

Further, even when the amount of the amide-containing cyclic sulfide compound was reduced to 5 mg or 2.5 mg, selective adsorption of palladium was observed. The palladium adsorption amount per 1 g of the amide-containing cyclic sulfide compound, when 2.5 mg of the amide-containing cyclic sulfide compound was used, was 0.8 mmol/g-Ligand. The above results (the relation between the amide-containing sulfide amount of the palladium ion adsorbent and the metal adsorption ratio) are shown in Fig. 1.

### EXAMPLE 2

Using 10 mg of the amide-containing cyclic sulfide compound prepared in Preparation Example 2 as the adsorbent, adsorption of palladium, platinum and rhodium was carried out in the same manner as in Example 1. As a result, the palladium adsorption ratio was 64.8%, the platinum adsorption ratio was 3.8%, and the rhodium adsorption ratio was 0%, thus indicating highly selective adsorption of palladium.

### EXAMPLE 3

Using 10 mg of the amide-containing cyclic sulfide compound prepared in Preparation Example 3 as the adsorbent, adsorption of palladium, platinum and rhodium was carried out in the same manner as in Example 1. As a result, the palladium adsorption ratio was 80.4%, the platinum adsorption ratio was 4.6%, and the rhodium adsorption ratio was 0%, thus indicating highly selective adsorption of palladium.

### EXAMPLE 4

Using 10 mg of the amide-containing cyclic sulfide compound prepared in Preparation Example 4 as the adsorbent, adsorption was carried out in the same manner as in Example 1. As a result, the palladium adsorption ratio was 57.0%, the platinum adsorption ratio was 0%, and the rhodium adsorption ratio was 0%, thus indicating highly selective adsorption of palladium.

### EXAMPLE 5

Using 10 mg of the amide-containing cyclic sulfide compound prepared in Preparation Example 5 as the adsorbent, adsorption was carried out in the same manner as in Example 1. As a result, the palladium adsorption ratio was 33.6%, the platinum adsorption ratio was 1.0%, and the rhodium adsorption ratio was 1.2%, thus indicating highly selective adsorption of palladium.

### EXAMPLE 6

Using 10 mg of the amide-containing cyclic sulfide compound prepared in Preparation Example 6 as the adsorbent, adsorption was carried out in the same manner as in Example 1. As a result, the palladium adsorption ratio was 48.4%, the platinum adsorption ratio was 1.6%, and the rhodium adsorption ratio was 0%, thus indicating highly selective adsorption of palladium.

### EXAMPLE 7

In a 50 mL eggplant flask, 0.2 g of the amide-containing cyclic sulfide compound prepared in Preparation Example 1 and 10 g of dimethyl sulfoxide (DMSO) were weighed, followed by stirring at 50°C for one hour. Then, 1.8 g of silica gel (manufactured by Wako Pure Chemical Industries, Ltd., trade name: Wakogel C-300) was added, followed by stirring at 50°C further for one hour. DMSO was distilled off under reduced pressure, and the obtained white powder was washed with water and dried under reduced pressure at room temperature to prepare silica gel having the amide-containing cyclic sulfide compound in a ratio of 10 wt% supported by impregnation. Using 100 mg (containing 10 mg of the amide-containing cyclic sulfide compound) of the silica gel as the palladium ion adsorbent, adsorption of palladium, platinum and rhodium was carried out in the same manner as in Example 1. As a result, the palladium adsorption ratio was 98.6%, the platinum adsorption ratio was 3.8%, and the rhodium adsorption ratio was 0%, thus indicating highly selective adsorption of palladium.

Further, even when the amount of the amide-containing cyclic sulfide compound fixed (supported) in 100 mg of the palladium ion adsorbent was reduced to 5 mg or 2.5 mg, selective adsorption of palladium was observed. That is, the palladium adsorption amount per 1 g of the amide-containing cyclic sulfide compound, when 2.5 mg of the amide-containing cyclic sulfide compound was used, was 1.5 mmol/g-Ligand. The above results (the relation between the amide-containing sulfide amount of the palladium ion adsorbent and the metal adsorption ratio) are shown in Fig. 2.

### EXAMPLE 8

Following the metal adsorption test procedure using 100 mg of the palladium ion adsorbent in Example 7, 10 mg of the palladium ion adsorbent in which palladium and platinum were adsorbed, after use, was stirred in 10 mL of each of eluents as identified in Table 1 at room temperature for one hour so that the metals are eluted in an aqueous layer. Then, the aqueous layer was subjected to filtration through a membrane filter having a pore size of 0.45 µm, and the eluted metal concentration in the filtrate was measured by an ICP emission spectrometer (manufactured by PerkinElmer Co., Ltd., trade name: OPTIMA 3300 DV). The elution ratio of each metal was determined from the eluted metal concentration and the initial concentration before stirring. The results are shown in Table 1.

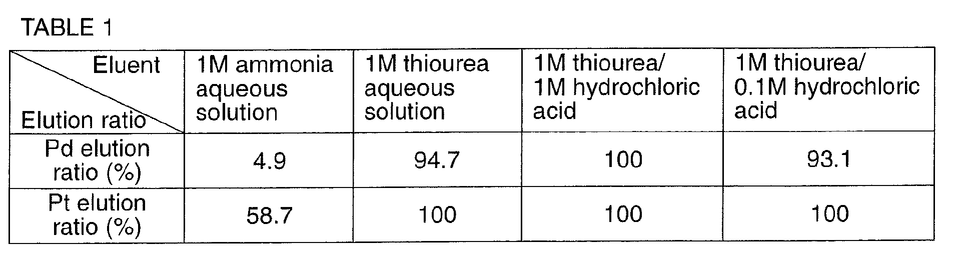

As evident from Table 1, palladium and platinum were eluted quantitatively when a 1 mol/L thiourea/1 mol/L hydrochloric acid solution was used as the eluent.

### EXAMPLE 9

In a 50 mL eggplant flask, 0.1 g of the amide-containing cyclic sulfide compound prepared in Preparation Example 7 and 10 g of tetrahydrofuran (THF) were weighed, followed by stirring at 40°C for 30 minutes. Then, 0.9 g of silica gel (manufactured by FUJI SILYSIA CHEMICAL LTD., trade name: SMB 300-10) was added, followed by stirring at 40°C further for 30 minutes. Then, THF was distilled off under reduced pressure, and the obtained white powder was dried under reduced pressure at room temperature to prepare silica gel having the amide-containing cyclic sulfide compound in a ratio of 10 wt% supported by impregnation.

Using 25 mg (containing 2.5 mg of the amide-containing cyclic sulfide compound) of the silica gel prepared above as the palladium ion adsorbent, adsorption of palladium, platinum and rhodium was carried out in the same manner as in Example 1. As a result, the palladium adsorption ratio was 73.3%, and the platinum adsorption ratio and the rhodium adsorption ratio were 0%, thus indicating highly selective adsorption of palladium. In this Example, the palladium adsorption amount per 1 g of the amide-containing cyclic sulfide was 1.4 mmol/g-Ligand.

### EXAMPLE 10

In a 50 mL eggplant flask, 0.1 g of the amide-containing cyclic sulfide compound prepared in Preparation Example 7 and 10 g of tetrahydrofuran (THF) were weighed, followed by stirring at 40°C for 30 minutes. Then, 0.9 g of alumina (manufactured by Wako Pure Chemical Industries, Ltd., trade name: Active alumina) was added, followed by stirring at 40°C further for 30 minutes. Then, THF was distilled off under reduced pressure, and the obtained white powder was dried under reduced pressure at room temperature to prepare alumina having the amide-containing cyclic sulfide compound in a ratio of 10 wt% supported by impregnation.

In order to make the alumina prepared above adsorb palladium, 400 mg (containing 40 mg of the amide-containing cyclic sulfide compound) of the alumina was added, as the palladium ion adsorbent, to 10 mL of a 1 mmol/L hydrochloric acid solution containing palladium with a concentration of 500 mg/L, followed by stirring at room temperature for 2 hours.

40 mg (palladium content: 12.5 mg/g) of the alumina obtained above in which palladium was adsorbed was stirred in 10 mL of each of eluents as identified in Table 2 at room temperature for one hour to elute palladium in an aqueous layer. Then, the aqueous layer was subjected to filtration through a membrane filter having a pore size of 0.45 µm, and the eluted metal concentration in the filtrate was measured by an ICP emission spectrometer (manufactured by PerkinElmer Co., Ltd., trade name: OPTIMA 3300 DV). The elution ratio of palladium metal was determined from the eluted metal concentration and the initial metal concentration. The results are shown in Table 2.

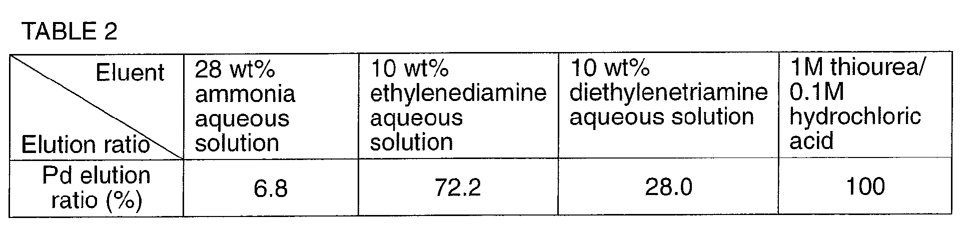

As evident from Table 2, palladium was efficiently eluted when a 1 mol/L thiourea/1 mol/L hydrochloric acid solution or a 10 wt% ethylenediamine aqueous solution was used as the eluent.

### EXAMPLE 11

In a 50 mL eggplant flask, 0.1 g of the amide-containing cyclic sulfide compound prepared in Preparation Example 8, 9 mL of chloroform and 1 mL of methanol were weighed, followed by stirring at 40°C for 30 minutes. Then, 0.9 g of silica gel (manufactured by FUJI SILYSIA CHEMICAL LTD., trade name: MB5D 200-350) was added, followed by stirring at 40°C further for 30 minutes. Then, THF was distilled off under reduced pressure, and the obtained white powder was dried under reduced pressure at room temperature to prepare silica gel having the amide-containing cyclic sulfide compound in a ratio of 10 wt% supported by impregnation.

Using 25 mg (containing 2.5 mg of the amide-containing cyclic sulfide compound) of the silica gel prepared above as the palladium ion adsorbent, adsorption of palladium, platinum and rhodium was carried out in the same manner as in Example 1. As a result, the palladium adsorption ratio was 94.3%, the platinum adsorption ratio was 2.9% and the rhodium adsorption ratio was 0%, thus indicating highly selective adsorption of palladium. In this Example, the palladium adsorption amount per 1 g of the amide-containing cyclic sulfide was 1.8 mmol/g-Ligand.

### EXAMPLE 12

In a 50 mL eggplant flask, 0.1 g of the amide-containing cyclic sulfide compound prepared in Preparation Example 9, 8 mL of chloroform and 2 mL of methanol were weighed, followed by stirring at 40°C for 30 minutes. Then, 0.9 g of silica gel (manufactured by FUJI SILYSIA CHEMICAL LTD., trade name: MB5D 200-350) was added, followed by stirring at 40°C further for 30 minutes. Then, THF was distilled off under reduced pressure, and the obtained white powder was dried under reduced pressure at room temperature to prepare silica gel having the amide-containing cyclic sulfide compound in a ratio of 10 wt% supported by impregnation.

Using 25 mg (containing 2.5 mg of the amide-containing cyclic sulfide compound) of the silica gel prepared above as the palladium ion adsorbent, adsorption of palladium, platinum and rhodium was carried out in the same manner as in Example 1. As a result, the palladium adsorption ratio was 99.7%, the platinum adsorption ratio was 17.6% and the rhodium adsorption ratio was 0%, thus indicating highly selective adsorption of palladium. In this Example, the palladium adsorption amount per 1 g of the amide-containing cyclic sulfide was 1.9 mmol/g-Ligand.

### EXAMPLE 13

In a 50 mL eggplant flask, 0.27 g of the amide-containing cyclic sulfide compound prepared in Preparation Example 7 and 10 g of tetrahydrofuran (THF) were weighed, followed by stirring at 40°C for 30 minutes. Then, 0.63 g of silica gel (manufactured by FUJI SILYSIA CHEMICAL LTD., trade name: CARiACT Q-50) was added, followed by stirring at 40°C further for 30 minutes. Then, THF was distilled off under reduced pressure, and the obtained white powder was dried under reduced pressure at room temperature to prepare silica gel having the amide-containing cyclic sulfide compound in a ratio of 30 wt% supported by impregnation.

0.3 g of the silica gel prepared above was dispersed in water and packed in a column made of glass having an inner diameter of 5 mm and a length of 100 mm. 75 mL of a 1 mol/L hydrochloric acid solution containing palladium with a concentration of 300 ppm was passed from the top of the column at a flow rate of 36 mL/h to carry out palladium adsorption, and the palladium concentration in the eluent eluted from the bottom of the column was measured by an ICP emission spectrometer (manufactured by PerkinElmer Co., Ltd., trade name: OPTIMA 3300 DV) to calculate the palladium adsorption amount. 20 mL of water was passed to rinse the column, and then 40 mL of a 1 mol/L hydrochloric acid solution containing thiourea with a concentration of 1 mol/L was passed from the top of the column at a flow rate of 36 mL/h to carry out palladium desorption, and the palladium concentration in the eluent eluted from the bottom of the column was measured by an ICP emission spectrometer (manufactured by PerkinElmer Co., Ltd., trade name: OPTIMA 3300 DV) to calculate the palladium desorption amount. Then, 20 mL of water was passed to rinse the column. The above operation of adsorption and desorption of palladium as one cycle was repeatedly carried out ten times to evaluate the durability of the adsorbent. The results are shown in Table 3.

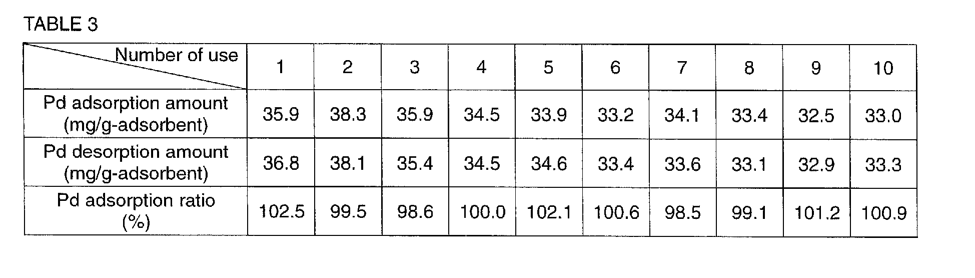

As evident from Table 3, the decrease in the palladium adsorption amount by repeated use is small, and even at the time of tenth use, a palladium adsorption amount of about 92% of the first use was observed. Further, the palladium desorption ratio (a percentage of the palladium desorption amount to the palladium adsorption amount) was almost 100% each time.

### INDUSTRIAL APPLICABILITY

The palladium ion adsorbent of the present invention has high affinity with palladium and highly selectively adsorbs palladium ions. Further, from the eluent obtained in the present invention, palladium adsorbed in the adsorbent can efficiently be recovered. Thus, the methods for separating and recovering palladium of the present invention are industrially useful since palladium in industrial catalysts and automobile exhaust gas purifying catalysts can efficiently be adsorbed and recovered without use of an organic solvent.

The entire disclosures of Japanese Patent Application No. 2009-192466 filed on August 21, 2009 and Japanese Patent Application No. 2009-192467 filed on August 21, 2009 including specifications, claims, drawings and summaries are incorporated herein by reference in their entireties.

## Claims

1. A palladium ion adsorbent, comprising an amide-containing cyclic sulfide compound represented by the following formula (1): 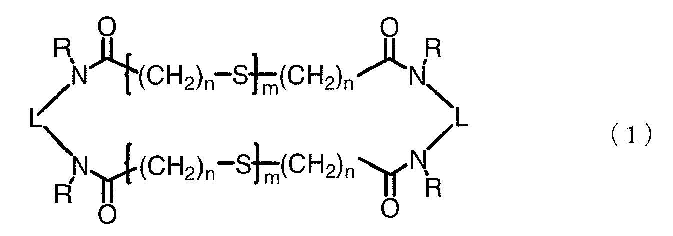 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, m is an integer of 1 or 2, n is each independently an integer of from 1 to 12, and L is each independently a methylene group, an ethylene group, a C₃₋₈ alkylene group or a C₆₋₁₄ arylene group.

2. The palladium ion adsorbent according to Claim 1, wherein in the formula (1), R is each independently a hydrogen atom, a methyl group, an ethyl group or a C₃-₁₈ linear or branched chain hydrocarbon group, and n between the carbonyl group and the sulfur atom is each independently an integer of from 1 to 4.

3. A palladium ion adsorbent, comprising an amide-containing cyclic sulfide compound represented by the following formula (2): 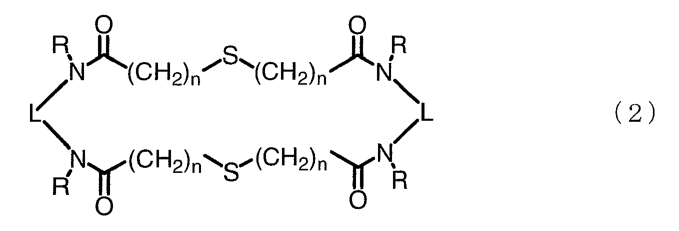 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, n is each independently an integer of from 1 to 4, and L is each independently a methylene group, an ethylene group, a C₃-₈ alkylene group or a C₆₋₁₄ arylene group.

4. The palladium ion adsorbent according to Claim 3, wherein in the formula (2), R is each independently a hydrogen atom, a methyl group, an ethyl group or a C₃-₁₈ linear or branched chain hydrocarbon group.

5. A palladium ion adsorbent, having an amide-containing cyclic sulfide compound represented by the following formula (1) or an amide-containing cyclic sulfide compound represented by the following formula (2) fixed on a carrier: 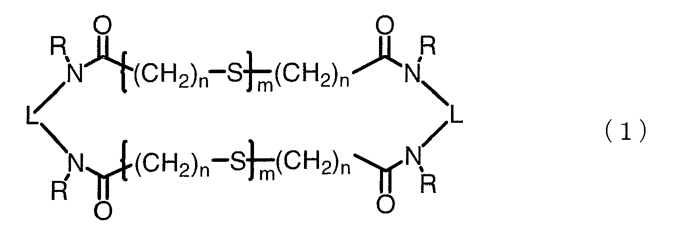 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, m is an integer of 1 or 2, n is each independently an integer of from 1 to 12, and L is each independently a methylene group, an ethylene group, a C₃₋₈ alkylene group or a C₆₋₁₄ arylene group; 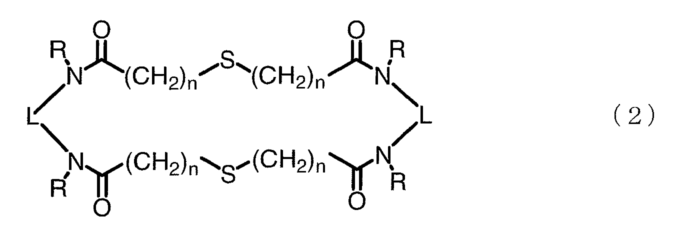 wherein R is each independently a hydrogen atom, a methyl group, an ethyl group, a C₃₋₃₀ linear or branched chain hydrocarbon group, a C₃₋₁₀ alicyclic hydrocarbon group or a C₆₋₁₄ aromatic hydrocarbon group, n is each independently an integer of from 1 to 4, and L is each independently a methylene group, an ethylene group, a C₃₋₈ alkylene group or a C₆₋₁₄ arylene group.

6. The palladium ion adsorbent according to Claim 5, wherein the carrier is silica gel.

7. The palladium ion adsorbent according to Claim 5, wherein in the formula (1), R is each independently a hydrogen atom, a methyl group, an ethyl group or a C₃₋₁₈ linear or branched chain hydrocarbon group, and n between the carbonyl group and the sulfur atom is each independently an integer of from 1 to 4.

8. The palladium ion adsorbent according to Claim 5, wherein in the formula (2), R is each independently a hydrogen atom, a methyl group, an ethyl group or a C₃-₁₈ linear or branched chain hydrocarbon group.

9. A method for separating palladium, which comprises bringing the palladium ion adsorbent as defined in any one of Claims 1 to 8 into contact with an aqueous solution containing palladium to make palladium be adsorbed in the palladium ion adsorbent.

10. A method for recovering palladium, which comprises bringing the palladium ion adsorbent as defined in any one of Claims 1 to 8 into contact with an aqueous solution containing palladium to make palladium be adsorbed in the palladium ion adsorbent, and then eluting palladium adsorbed in the palladium ion adsorbent by an eluent to obtain an aqueous solution containing palladium.
